Europäisches Patentamt

European Patent Office

Office européen des brevets

(19)

(11) Publication number: **0 304 619 B1**

(12) # EUROPEAN PATENT SPECIFICATION

(45) Date of publication of patent specification: **16.10.91**  (51) Int. Cl.5: **A61M 5/32, A61M 5/00**

(21) Application number: **88111643.8**

(22) Date of filing: **20.07.88**

(54) Injection needle-detaching device and container having said device.

(30) Priority: **31.07.87 JP 117564/87 U**
**14.12.87 JP 189702/87 U**

(43) Date of publication of application:
**01.03.89 Bulletin 89/09**

(45) Publication of the grant of the patent:
**16.10.91 Bulletin 91/42**

(84) Designated Contracting States:
**DE IT NL**

(56) References cited:
**EP-A- 0 136 392**
**WO-A-82/00412**
**WO-A-88/00067**
**DE-U- 8 614 635**
**DE-U- 8 708 179**

(73) Proprietor: **NISSHO CORPORATION**
**9-3, Honjo-nishi, 3-chome Oyodo-ku**
**Osaka-shi(JP)**

(72) Inventor: **Yoshida, Toshiki**
**12-3-508, Aoba 3-chome**
**Shimamoto-cho Mishima-gun Osaka-fu(JP)**
Inventor: **Nakamura, Motohiko**
**Daisan Iwashiba Ryo 1-4-5, Iwase**
**Kamakura-shi Kanagawa-ken(JP)**

(74) Representative: **Türk, Gille, Hrabal**
**Brucknerstrasse 20**
**W-4000 Düsseldorf 13(DE)**

## Description

The present invention relates to a container having an injection needle detaching means for containing used injection needles, having the features of the pre-characterizing part of claim 1, and to a device for detaching an injection needle from a syringe, having the features of the pre-characterizing part of claim 3.

There are two kinds of injection needles with respect to the manner of attaching the needle to a syringe. The one is a slip type wherein a needle has a hub with a plain inner surface and the hub is simply forced to be put on the nozzle portion of a syringe that has a plain outer surface. The other is a screw type wherein a needle has a hub having a female thread on the inner surface thereof and the hub is threaded onto the nozzle portion of a syringe that has a male thread on the outer surface thereof.

Conventionally, the injection needle was detached from the syringe by simply pulling out it or by unthreading it with holding the hub thereof with fingers. At the first glance, such detaching operation appears to be easy. However, an accident that finger is injured with the needle inevitably happens when the same person repeats the needle-detaching operation several tens times or more a day.

Recently diseases infectious through blood such as viral hepatitis and acquired immuno-deficiency syndrome (AIDS) have increased rapidly. For the reason, it is important that persons who are engaged in test for the diseases and treatment of the diseases get out of direct touch with the blood of patients.

However, when a used needle is detached from a syringe in the conventional manner, there is a great possibility that a person gets hurt in the finger with the needle and the blood attached to the needle is entered into the body of the person through the wound so that the person is infected with the above-mentioned disease.

Accordingly, there is a demand for a means of detaching the needle from the syringe without touching the needle with finger.

With respect to blood-drawing needle which generally has two needles extending in the opposed directions from the hub, a needle-receiving container in compliance with the demand is proposed, as disclosed in United States Patent No. 4,466,538. The container is provided with a slot which has a large width at one end and is narrowed toward the other end. A blood-drawing needle attached to a syringe is inserted into the wide portion of the slot and then moved toward the narrow portion, thereby catching the hub of the needle between both the side walls of the narrow portion. When the syringe is turned in such a state, the engagement between the syringe and the needle is loosened, so that the needle falls into the container.

However, the container has a drawback that since the hub of the needle is firmly caught between the side walls when the syringe is turned, the needle tends to remain in that position without falling into the container and the operator gets hurt in the finger with the needle when he touches the needle to remove the caught needle.

A container having an injection needle detaching means for containing used injection needles as disclosed in EP-A 0 136 392 comprises a closed container for containing injection needles and a needle-detaching means for detaching a slip type injection needle from a syringe, said needle-detaching means comprising a first opening provided in a wall of said container for inserting said injection needle attached to said syringe and a second opening extending laterally from said first opening in a slit-like manner. The first opening has a size larger than the largest part of the needle, the second opening has a width larger than the diameter of the nozzle of the syringe and smaller than the diameter of the hub of said needle, so that the needle, after being inserted into the first opening, can be moved along the second opening, below which a supporting frame is provided, to detach the needle from the syringe. For enabling the detaching, the supporting frame inclines in direction of the slit-like second opening, and the needle is urged away from the syringe.

However, the handling of the known device is not reliable in the case that a large number of syringes have to be treated. In the initial stage of the detaching operation the hub of the needle cannot be caught surely by the inclined surface of the supporting frame, so attention is required when trying to proceed the syringe along the second opening. If an operator has to repeat the detaching many times a day his concentration will decrease and accidents may occur.

Further, the known device is only suitable for slip type needles, it is not possible to detach screw type needles.

It is the object of the present invention to provide a needle-detaching device for a slip type needle capable of detaching a used needle from a syringe with an easy operation without touching the needle with finger.

Further object of the invention is to provide a needle-detaching device having both a needle-detaching function for a slip type needle and a needle-detaching function for a screw type needle.

Still further object of the invention is to provide a container equipped with the above-mentioned needle-detaching device and for detaching a used needle from a syringe and containing the detached needle directly therein.

These and other objects of the present invention will become apparent from the description hereinafter.

This object is achieved by a a device for detaching an injection needle of the kind described in the introduction having the features of the characterizing part of patent claim 1. Preferred embodiments are given in claims 2 to 4, respectively.

Fig. 1 is a perspective view showing a container for containing used injection needles equipped with an embodiment of the needle-detaching device of the present invention.

Fig. 2 is a partially broken, enlarged perspective view showing an example of the needle-detaching means of the device shown in Fig. 1, viewed from the inside of the container.

Fig. 3 is a partially broken, enlarged perspective view showing another example of the needle-detaching means, viewed from the inside of the container.

Fig. 4 is a partial sectional view showing a state wherein a syringe is inserted in the needle-detaching means shown in Fig. 2, corresponding to a section taken along the line I-I in Fig. 1.

Fig. 5 is a partial sectional view showing the same state as illustrated in Fig. 4, but corresponding to a section taken along with the line II-II.

Fig. 6 is a partial sectional view showing a state wherein a syringe is inserted in the needle-detaching means shown in Fig. 3, corresponding to a section taken along the line I-I in Fig. 1.

Fig. 7 is a partial sectional view showing the same state as illustrated in Fig. 6, but corresponding to a section taken along the line II-II in Fig. 1.

Fig. 8 is a perspective view showing another embodiment of the needle-detaching device of the present invention.

Fig. 9 is a plan view showing the device illustrated in Fig. 8.

Fig. 10 is an elevational view showing the device illustrated in Fig. 8.

Fig. 11 is a sectional view taken along the line III-III in Fig. 9.

Fig. 12 is a sectional view taken along the line IV-IV in Fig. 9.

Fig. 13 is a sectional view taken along the line V-V in Fig. 9.

Fig. 14 is a perspective view showing the needle-detaching device illustrated in Fig. 8 and a container on which the needle-detaching device is to be mounted.

Fig. 15 is an explanatory view showing the needle-detaching operation with respect to a slip type needle.

Fig. 16 is an explanatory view showing the needle-detaching operation with respect to a screw type needle.

Fig. 17 is an explanatory view showing a syringe.

First a syringe and an injection needle are explained in order to give a better understanding of the description hereinafter.

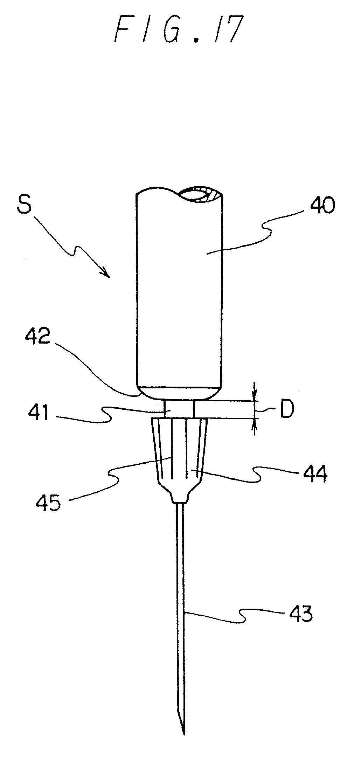

Referring to Fig. 17, the numeral 40 indicates a barrel of a syringe S. The syringe barrel 40 has a nozzle 41 at one end. The numeral 42 indicates a shoulder portion at the root of the nozzle 41. The numeral 43 indicates an injection needle and the injection needle 43 extends from a hub 44.

The above-mentioned elements are fundamental elements common to both a slip type needle and a screw type needle. In the case of the slip type needle, the hub 44 has a cavity with a plain inner surface and the corresponding nozzle 41 also has a plain outer surface. In the case of the screw type needle, the hub 44 has an inner surface provided with a female thread and the corresponding nozzle 41 has an outer surface provided with a male thread. Plural ribs 45 are provided on the outer surface of the hub 44 in the longitudinal direction thereof for the purpose of providing a moderate fitting strength between the hub 44 and a protector for the needle 43.

In the present invention, the term "syringe" is intended to comprehend a blood-collecting tube in addition to a normal syringe. Also the term "injection needle" is intended to comprehend a blood-drawing needle in addition to a normal injection needle.

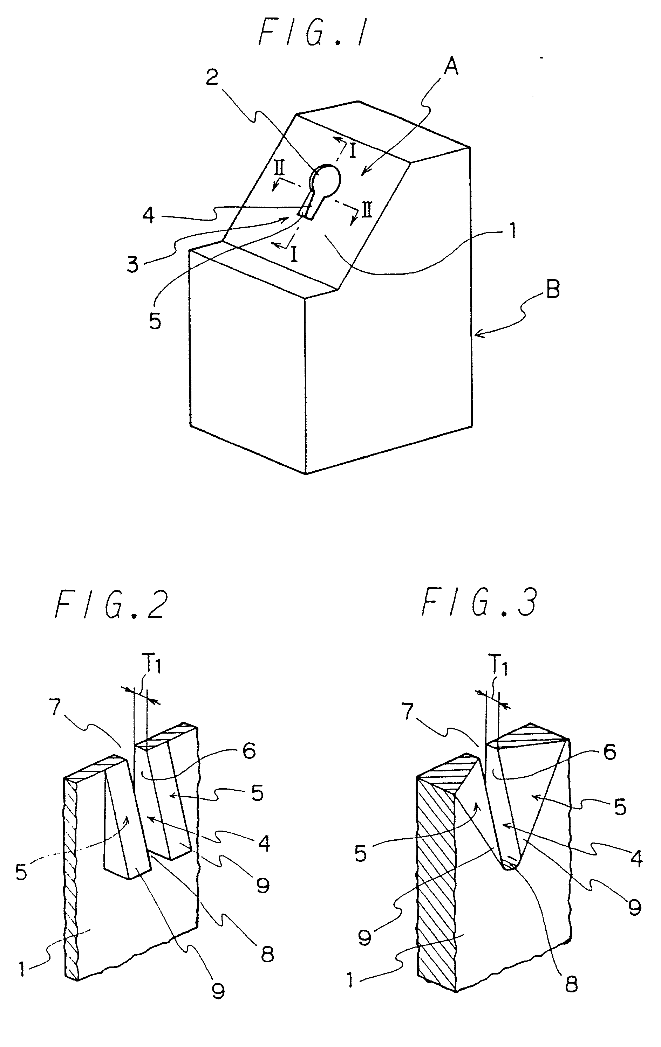

Referring to Figs. 1 to 7, an embodiment of a needle-detaching device for the slip type needle in accordance with the present invention will be explained.

Fig. 1 shows a container B for used needles in which a needle-detaching device A is provided.

The device A is mounted on an inclined plane of the container B. Needles detached from syringes by means of the device A are contained within the container B.

The construction of the container B is not limited to that illustrated in Fig. 1. The container B is made of wood, plastics, metal, etc. In the example shown in Fig. 1, the needle-detaching device A is installed only on one surface of the container B. However, a plurality of the device A may be installed on two or more surfaces of the container B.

The device A comprises a plate-like member 1, a first opening 2 for inserting an injection needle provided in the plate-like member 1 and a first needle-detaching means 3 for a slip type needle.

The needle-inserting opening 2 has a size larger than the largest part of an injection needle 43. Usually the largest part is a hub 44. However, when a protector is put on the needle 43, the largest part is the protector if the diameter of the protector is larger than that of the hub 44. Preferably the opening 2 has a size smaller than the diameter of the barrel 40 of the syringe S.

The first needle-detaching means 3 comprises a second opening 4 extending laterally from the needle-inserting opening 2 and communicating with the opening 2, and a sliding portion 5 provided on each of both sides of the second opening 4.

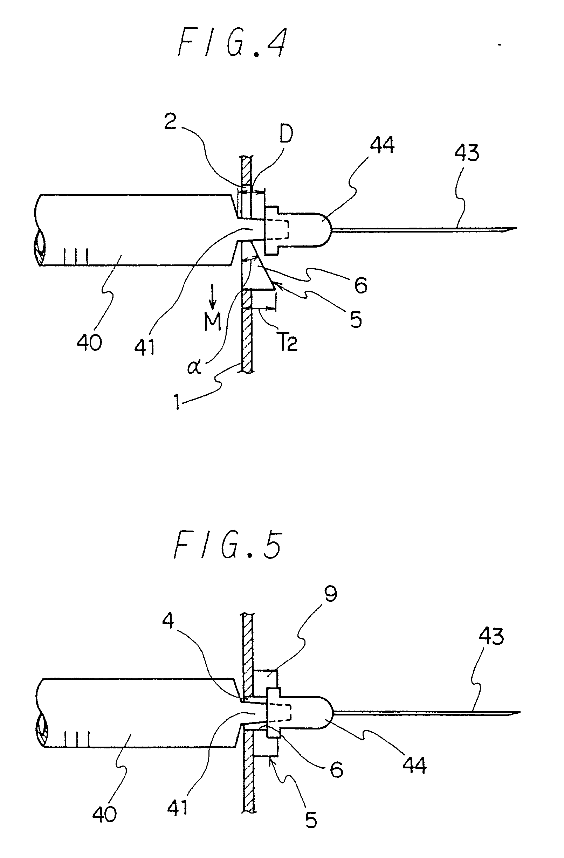

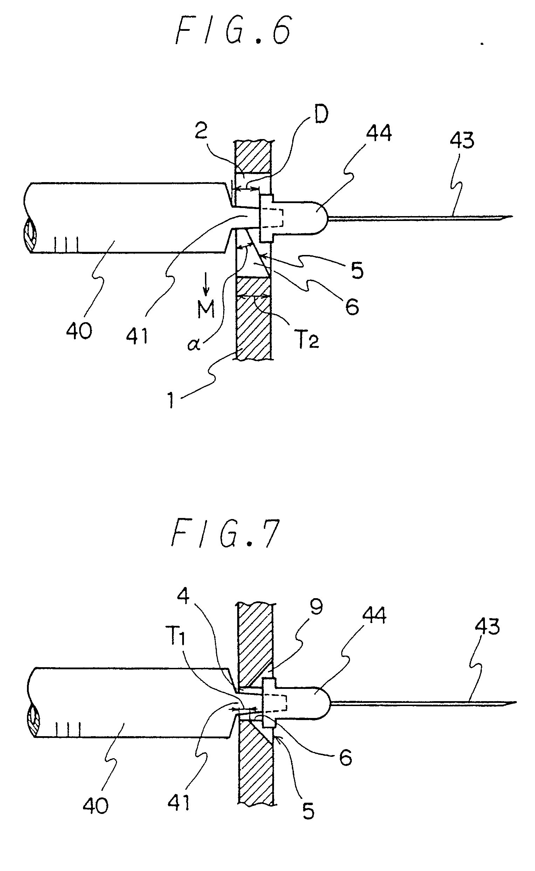

The second opening 4 has a width larger than the diameter of the nozzle 41 of the syringe S and smaller than the diameter of the hub 44, as shown in Fig. 5 and Fig. 7.

Each of the sliding portions 5 has a side wall 6 defining the second opening 4.

Each side wall 6 has, at the entrance 7 of the second opening 4 from the first opening 2, a thickness $T_1$ smaller than a distance D between the root of the nozzle 41 and the bottom end of the hub 44 when the needle 43 is being firmly attached to the syringe S, and each side wall 6 is made gradually thicker toward the closed end 8 of the second opening 4 and the maximum thickness $T_2$ of each side wall 6 at the closed end 8 is larger than the distance D, as shown in Figs. 2, 3, 4 and 6.

The shape of the second opening 4 is usually a rectangle in plane as shown in Fig. 1. However, the width of the second opening 4 may be made smaller toward the closed end 8. Further, the length of the second opening 4 may be reduced so that the plane shape becomes similar to a regular square.

In the case of an example of the needle-detaching means 3 as shown in Figs. 2, 4 and 5, the side wall 6 has the same thickness $T_1$ as that of the plate-like member 1 at the entrance 7 but the remaining parts of the side wall 6 are thicker than the member 1. The surface 9 of the sliding portion 5 on the rear side of the member 1 constitutes a sliding surface on which the bottom surface of the hub 44 slides. Both sliding surfaces 9, 9 are flash with each other.

In the case of another example of the needle-detaching means 3 in Figs. 3, 6 and 7, the side wall 6 has the same thickness $T_2$ as that of the plate-like member 1 at the closed end 8 but the remaining parts of the side wall 6 are thinner than the member 1. The surface 9 of the sliding portion 5 on the rear side of the member 1 constitutes a sliding surface on which the bottom surface of the hub 44 slides. Each sliding surface 9 is inclined both in the direction of the first opening 2 and in the direction of the second opening 4.

The side wall 6 must have a maximum thickness $T_2$ sufficient to remove the hub 44 from the nozzle 41. The maximum thickness $T_2$ is larger than a distance D between the root of the nozzle 41 and the bottom surface of the hub 44 to surely detach the hub 44 from the nozzle 41.

Further the angle $\alpha$ between both edge lines of the side wall 6 in the longitudinal direction of the second opening 4 is preferably from 5° to 45°, more preferably from 15° to 30°, to facilitate a smooth removal of the hub 44 from the nozzle 41.

The needle-detaching operation using the needle-detaching device A shown in Fig. 2 will be explained by referring to Figs. 4 and 5.

Fig. 4 and Fig. 5 are a sectional view taken along the line I-I of Fig. 1 and a sectional view taken along the line II-II of Fig. 1, respectively, when the syringe S is positioned on the line II-II of Fig. 1.

The lower portion of the syringe S to which the needle 43 is attached is inserted into the first opening 2 until the shoulder portion 42 of the barrel 40 is brought into contact with the pheriphery of the first opening 2 and then moved into the second opening 4, followed by further movement toward the closed end 8. When the syringe S is moved toward the closed end 8 in the direction of the arrow M, both side walls 6, 6 are inserted into the spacing between the shoulder portion 42 of the barrel 40 and the bottom surface of the hub 44 like a wedge because of increasing thickness of each side wall 6. As a result, the hub 44 is pushed downward so that it is loosened and finally separated from the nozzle 41.

The needle-detaching operation using the needle-detaching device A shown in Fig. 3 will be explained by referring to Figs. 6 and 7.

Fig. 6 and Fig. 7 are a sectional view taken along the line I-I of Fig. 1 and a sectional view taken along the line II-II of Fig. 1, respectively, when the syringe S is positioned on the line II-II of Fig. 1.

The needle-detaching device A shown in Fig. 3 is preferably used when the thickness of the plate-like member 1 is larger than the distance D between the root of the nozzle 41 and the bottom surface of the hub 44.

The lower portion of the syringe S to which the needle 43 is attached is inserted into the first opening 2 until the shoulder portion 42 of the barrel 40 is brought into contact with the pheriphery of the first opening 2 and then moved into the second opening 4. Both side walls 6, 6 are inserted into the spacing between the shoulder portion 42 of the barrel 40 and the bottom surface of the hub 44 at the entrance 7. When the syringe S is further moved toward the closed end 8 of the second opening 4, the hub 44 is gradually spaced apart from the shoulder portion 42 of the barrel 40 and finally separated from the nozzle 41, the separated needle 43 falling within the container B.

When used needles are accumulated in the container B, the container B is closed and subjected to a discarding treatment such as burning.

The device A and the container B can be manufactured by a conventional method. For instance, when the material is plastics, they can be injection-molded.

By using the needle-detaching device A of the present invention, a needle to which blood is attached can be detached smoothly from a syringe with an easy operation without touching the needle with the hand.

Further, the side walls are inserted into the spacing between the shoulder portion of the barrel and the bottom surface of the hub like a wedge, so that the hub is spaced apart from the nozzle and finally separated from the nozzle. Accordingly, there does not occur the problem of the conventional device that since the needle is caught in the device because of twisting the hub thereof to loosen it, a person such as doctor must remove the caught needle from the device by touching it with the hand, and there is no danger of infection.

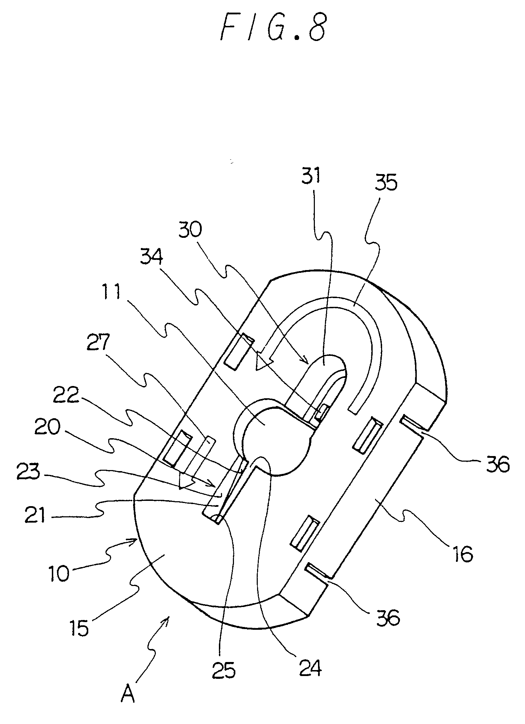

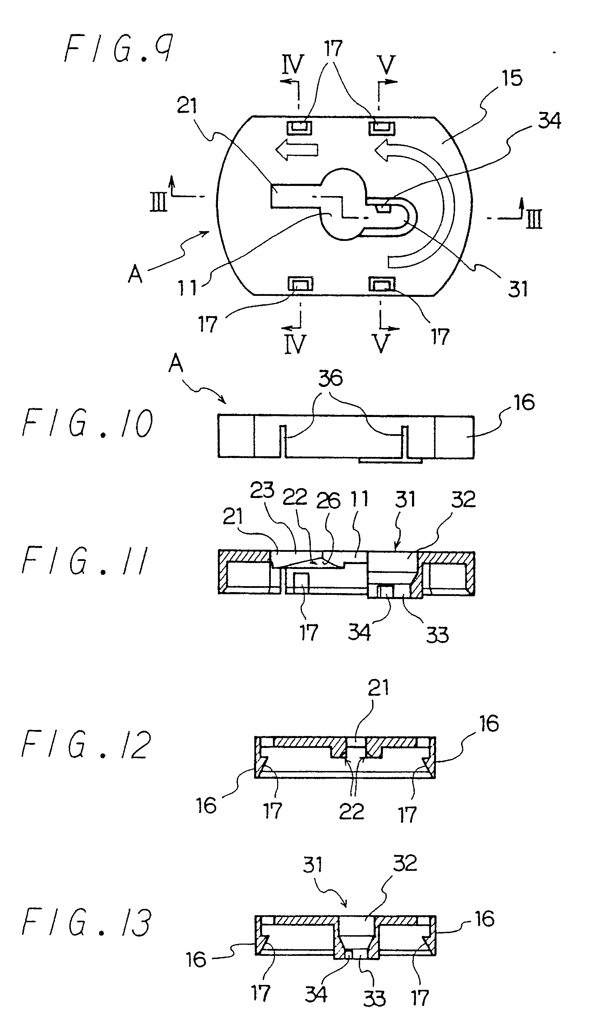

Another embodiment of the needle-detaching device of the present invention will be explained by referring to Figs. 8 to 16. The device of this embodiment has both the function of detaching a slip type needle and the function of detaching a screw type needle.

Fig. 8 illustrates a needle-detaching device A' in accordance with this embodiment. The needle-detaching device A' comprises a plate-like member 10, a first opening 11 for inserting the lower portion of a syringe S provided in the central portion of the member 10, a first needle-detaching means 20 for a slip type needle and a second needle-detaching means 30 for a screw type needle, the means 20 and the means 30 being provided on the opposed sides of the first opening 11.

The first needle-detaching means 20 has substantially the same construction and function as the above-mentioned first needle-detaching means 3.

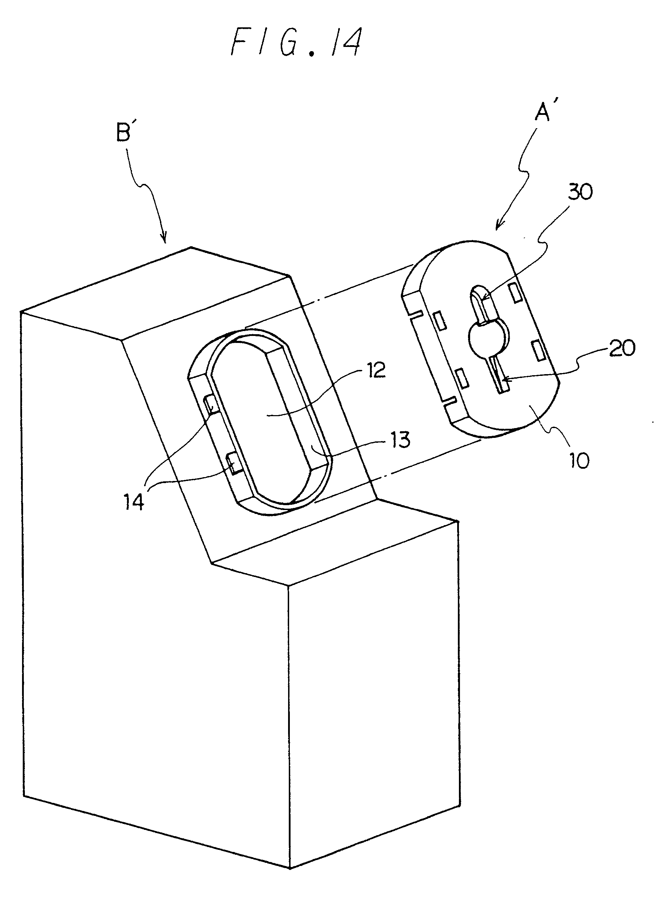

The needle-detaching device A', when being used, is mounted on a container B' for containing used needles, as shown in Fig. 14. The shape of the container B' is not particularly limited. An opening 12 is provided in a part of container B', and a rib 13 and engaging projections 14 are provided in the periphery of the opening 12 to install the needle-detaching device A'.

The construction of the needle-detaching device A' will be explained in detail by referring to Figs. 8 to 13.

The plate-like member 10 comprises an upper plate 15 and a skirt 16 provided in the periphery of the upper plate 15. The plate-like member 10 is integrally formed from a synthetic resin such as polyethylene, polypropyrene, acrylonitril-butadiene-styrene copolymer, polycarbonate or polyester.

The upper plate 15 has an opening 11 provided in the central portion thereof. The opening 11 is nearly elliptical in shape and has such a size that the hub 44 of the needle 43 can be inserted thereinto. When the needle 43 is covered with a protector, the opening 11 preferably has a size larger than the diameter of the protector so that the needle 43 covered with the protector can be detached from the syringe S.

The first needle detaching means 20 comprises a second opening 21 extending from and communicating with the first opening 11, and a sliding portion 22 provided on each of both sides of the second opening 21.

Each sliding portion 22 has a side wall 23 defining the second opening 21. Each sliding portion 22 is formed so that the side wall 23 is the thinnest at the entrance 24 of the second opening 21 and made gradually thicker toward the closed end 25 of the second opening 21.

The width of the second opening 21 is somewhat larger than the diameter of the nozzle 41 and must be smaller than that of the barrel 40 and that of the hub 44.

The thickness of the thinnest part of the side wall 23, i.e. the thickness of the side wall 23 at the entrance 24, must be smaller than the distance D between the root of the nozzle 41 and the bottom surface of the hub 44 when the hub 44 is firmly put on the nozzle 41. The thickest part of the side wall 23 must have a thickness sufficient to detach the hub 44 from the nozzle 41. The thickest part of the side wall 23 has a thickness larger than the distance D between the root of the nozzle 41 and the bottom surface of the hub 44 when the hub 44 is firmly put on the nozzle 41. The sliding surface 26 of the sliding portion 22 on the rear side of the plate 15, on which the bottom surface of the hub 44 slides, is inclined both in the direction of the first opening 11 and in the direction of the second opening 21.

Preferably an arrow mark 27 for indicating the movement direction of the syringe S is provided on a side of the second opening 21 on the plate 15.

The second needle-detaching means 30 contains a third opening 31 having a U shape in plain. The third opening 31 comprises a big-sized portion 32 having such a size that the hub 44 of the needle 43 is easily inserted thereinto and a small-sized portion 33 extending vertically from the big-sized portion 32 and having such a size that only the tip portion of the hub 44 can be inserted thereinto. The small-sized portion 33 has a projection 34 formed on a part of the side wall thereof. The projection 34 serves to prevent the rotation of the hub 44 due to the engagement of the projection 34 with the rib 45 of the hub 44.

The reason why the small-sized portion 33 is provided under the big-sized portion 32 and the projection 34 is provided in the small-sized portion 33 is that the center of gravity of the needle 43 when being inserted in the third opening 31 is positioned under the upper surface of the plate 15, thereby preventing the detached needle 43 from falling out of the device A'.

Preferably an arrow mark 35 for indicating the direction of the rotation of the syringe S is provided around the third opening 31.

The skirt 16 has two slits 36, 36 on each of the opposed sides thereof so that a part of the skirt 16 between the two slits 36, 36 is made somewhat flexible. The part of the skirt 16 between the two slits 36, 36 has engaging projections 17 on the rear surface thereof. The engaging projections 17 are engaged with the engaging projections 14 of the container B'. The number of the projections 17 are not particularly limited. In this example, two projections on each side of the skirt 16, four projections in total, are formed. The device A' is installed to the container B' by resiliently engaging the projections 14 of the container B' with the projections 17 of the device A'.

The operation of detaching an injection needle from a syringe by means of the device A' is explained.

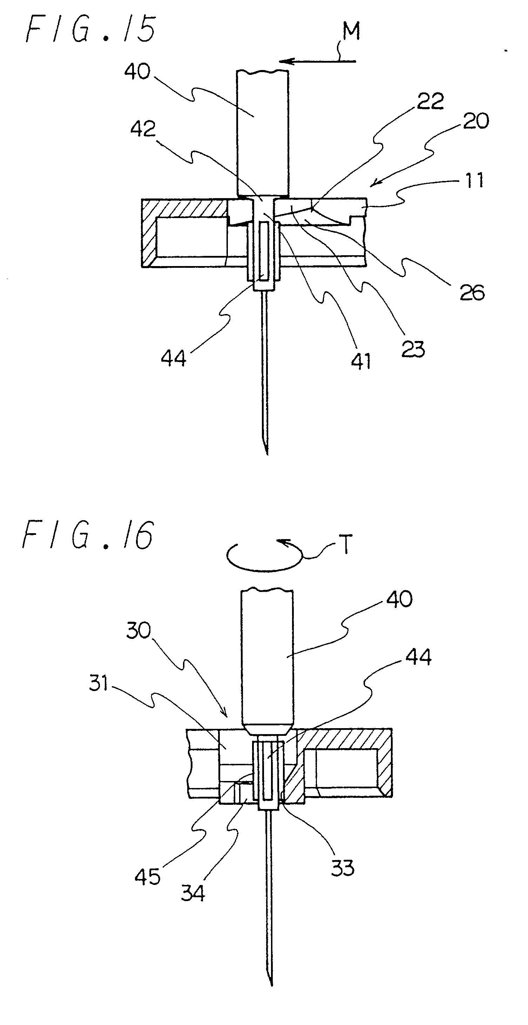

Fig. 15 illustrates the operation of detaching a slip type needle. The lower portion of the injection barrel 40 is inserted into the first opening 11 and put on the entrance 24 of the second opening 21. The nozzle 41 is inserted between both side walls 23, 23 and the shoulder portion 42 is brought into contact with the upper surfaces of the sliding portions 22 and the bottom surface of the hub 44 is brought into contact with the sliding surfaces 26, 26. When the injection barrel 40 is moved in the direction of the arrow M, the hub 44 is pushed downward because of increasing thickness of each sliding portion 22, thereby separating the hub 44 from the nozzle 41. The device A' is preferably installed to the container B' so that the barrel 40 can be moved downward as shown in Fig. 14 because it is easy to apply force on the barrel 40.

Fig. 16 illustrates the operation of detaching a screw type needle. The lower portion of the injection barrel 40 is inserted downward into the third opening 31 and the hub 44 is inserted into the small-sized portion 33, engaging the rib 45 of the hub 44 with the projection 34. When the barrel 40 is turned several turns in the direction of the arrow T in such a state, the needle 43 is loosened and detached from the nozzle 41. Alternatively the barrel 40 is turned about one turn, followed by movement toward the first opening 11. In that case, the hub 44 is turned in such a manner that it crosses the projection 34, whereby the hub 44 is unthreaded and detached from the nozzle 41.

As described above, by using the needle-detaching device A', an injection needle can be detached without touching the tip of the injection needle with finger. Further an injection needle can be detached by using only the device A' regardless of whether the needle is a slip type needle or a screw type needle.

The device A' of the above-mentioned example is nearly elliptical in plane shape. However the device A' may be circular in plane shape. In that case, the skirt 16 can be designed so that it can be installed to a container by fitting or screwing. The device A' having such a skirt 16 can be installed to a suitable bottle as the container B'. In that case, no individually designed container is needed.

By using the needle-detaching device A' of the present invention, an injection needle can be readily detached with safety without injuring the finger. Further detachment of both a slip type needle and a screw type needle can be effected by using only the device A'.

In addition to the elements used in the Examples, other elements can be used in the Examples as set forth in the specification and the drawings to obtain substantially the same results.

## Claims

1. A device for detaching an injection needle from a syringe comprising:
   a plate-like member (10),
   a first opening (11) provided in the plate-like member (10) for inserting said injection needle (43) attached to said syringe (S),
   a first needle-detaching means (20) for detaching a slip type needle,
   said first opening (11) having a size larger than the largest part of said needle (43),
   said first needle-detaching means (20) comprising a second opening (21) extending laterally from said first opening (11),
   said second opening (21) having a width larger than the diameter of the nozzle (41) of said syringe (S) and smaller than the diameter of the hub (44) of said needle (43),
   said first needle-detaching means (20) comprising a sliding portion (22) provided on each of both sides of said second opening (21),
   each of said sliding portions (22) having a side wall (23) defining said second opening (21),
   each of said side walls (23) having, at the entrance (24) of the second opening (21) from said first opening (11), a thickness smaller than a distance (D) between the root of the nozzle (41) of said syringe (S) and the bottom end of the hub (44) of said needle (43) when the needle (43) is being attached to said syringe (S), each of said side walls (23) being made gradually thicker towards the closed end (25) of said second opening (21), the maximum thickness of each of said side walls (23) being larger than said distance (D), characterized in that

the surface (26) of said sliding portion (22) on the rear side of said plate-like member (10), on which the bottom surface of the hub (44) slides, forms a surface inclined both in the direction of the first opening (11) and in the direction of the second opening (21), and in that

a second needle-detaching means (30) for detaching a screw type needle is provided, said second needle-detaching means (30) comprising a third opening (31) extending laterally from said first opening (11), said third opening (31) having a portion (32) having such a size that the hub (44) of said needle (43) is capable of being inserted thereinto, said portion (32) being provided with a projection (34) for preventing the hub (44) of said needle (43) from turning when the syringe (S) is turned.

2. The device as claimed in claim 1, characterized in that the angle between both edge lines of each side wall (23) in the longitudinal direction of said second opening (21) is from 5° to 45°.

3. The device as claimed in claim 1, characterized in that said third opening (31) comprises a big-sized portion (32) having such a size that the hub (44) of the needle (43) is smoothly inserted thereinto and a small-sized portion (33) extending vertically from the big-sized portion (32) and having such a size that only the tip portion of the hub (44) is capable of being inserted thereinto, said projection (34) being provided on a part of the side wall of the small-sized portion (33).

4. The device as claimed in claim 1, characterized in that said plate-like member (10) is designed so that it is detachably installed to a container for containing removed injection needles.

5. A container for containing used injection needles comprising:
a container (B), and
a needle-detaching device as recited in any of the preceding Claims provided in a part of the container.

6. The container of Claim 5, in which the needle-detaching device is detachably installed to the container.

**Revendications**

1. Dispositif pour détacher une aiguille d'injection d'une seringue comprenant:
un élément sous forme de plaque (10),
une première ouverture (11) prévue dans ledit élément sous forme de plaque (10) pour insérer ladite aiguille d'injection (43) fixée à la seringue (S),
un premier dispositif (20) pour détacher une aiguille coulissante,
ladite première ouverture (11) ayant une dimension plus large que la partie la plus large de ladite aiguille (43),
ledit premier dispositif (20) pour détacher l'aiguille comprenant une deuxième ouverture (21) s'étendant latéralement de la première ouverture (11),
ladite deuxième ouverture (21) étant plus large que le diamètre de l'orifice (41) de la seringue (S) et plus petite que le diamètre du moyeu (44) de ladite aiguille (43),
ledit premier dispositif (20) pour détacher l'aiguille comprenant une partie coulissante (22) prévue de part et d'autre des deux cotés de ladite deuxième ouverture (21),
chacune desdites parties coulissantes (22) ayant une paroi latérale (23) définissant ladite deuxième ouverture (21),
chacune desdites parois laterales (23) ayant au passage (24) entre ladite deuxième ouverture (21) et ladite première ouverture (11) une épaisseur plus petite qu'une distance (D) entre la racine de l'orifice (41) de la seringue (S) et l'extrémité inférieure du moyeu (44) de l'aiguille (43) lorsque l'aiguille (43) est fixée à la seringue (s), l'épaisseur de chacune desdites parois latérales (23) augmentant progressivement en direction de l'extrémité fermée (25) de ladite deuxième ouverture (21), l'épaisseur maxi de chacune desdites parois latérales (23) étant plus grande que ladite distance (D),
**caractérisé en ce que**
la surface (26) de ladite partie coulissante (22) à la face arrière de l'élément sous forme de plaque (10), sur laquelle coulisse la face arrière du moyeu (44), forme une surface inclinée aussi bien dans la direction de la première ouverture (11) que dans la direction de la deuxième ouverture (21) et qu'il est prévu un deuxième dispositif (30) pour détacher une aiguille du type vissable,
ledit deuxième dispositif (30) pour détacher une aiguille comprenant une troisième ouverture (31) s'étendant latéralement de la première ouverture (11),
ladite troisième ouverture (31) comprenant une partie (32) de dimension telle que le moyeu (44) de ladite aiguille (43) puisse y être insérée, ladite partie (32) étant pourvue d'une projection (34) afin d'éviter que le moyeu (44) de l'aiguille (43) ne tourne lorsque la séringue (S) est tournée.

2. Dispositif suivant la revendication 1, **caractérisé en ce que** l'angle entre les deux bords de chacune des parois latérales (23) dans la direction latérale de ladite deuxième ouverture (21) se situe dans la gamme de 5° à 45°.

3. Dispositif suivant la revendication 1, **caractérisé en ce que** ladite troisième ouverture (31) comprend une partie de grande dimension (32) ayant une dimension telle que le moyeu (44) de l'aiguille (43) puisse facilement y être insérée, ainsi qu'une partie (33) de petite dimension s'étendant verticalement de la partie à grande dimension (32) et ayant une dimension telle que seulement la partie supérieure du moyeu (44) puisse y être insérée, ladite projection étant prévue sur une partie de la paroi latérale de la partie à petite dimension (33).

4. Dispositif suivant la revendication 1, **caractérisé en ce que** ledit élément sous forme de plaque (10) est conçu tel qu'il est installé de manière détachable dans un conteneur recevant des aiguilles utilisées.

5. Conteneur pour recevoir des aiguilles utilisées comprenant:
un conteneur (B), et
un dispositif pour détacher les aiguilles d'injection suivant l'une des revendications précédentes et qui est prévu dans une partie du conteneur.

6. Conteneur suivant la revendication 5, dans lequel le dispositif pour détacher les aiguilles d'injection est installé de façon détachable dans le conteneur.

**Patentansprüche**

1. Vorrichtung für das Abziehen einer Injektionsnadel von einer Spritze, die folgendes aufweist:
einen plattenförmigen Teil (10)
eine erste Öffnung (11) in dem plattenförmigen Teil (10), um die an der Spritze (S) befestigte Injektionsnadel (43) einzuführen,
eine erste Nadelabzugsvorrichtung (20), um eine einschiebbare Nadel abzuziehen,
wobei diese erste Öffnung (11) eine größere Abmessung hat, als der breiteste Teil der Nadel (43),

und die erste Nadelabzugsvorrichtung (20) eine sich seitlich von der ersten Öffnung (11) erstreckende zweite Öffnung (21) aufweist,
wobei diese zweite Öffnung (21) eine Weite hat, die größer ist als der Durchmesser des Mundstückes (41) der Spritze (S) und kleiner ist, als die Nabe (44) der Nadel (43),
und die erste Nadelabzugsvorrichtung (20) einen Gleitteil (22) an beiden Seiten der zweiten Öffnung (21) aufweist,
wobei jeder der Gleitteile (22) eine Seitenwand (23) aufweist, die die zweite Öffnung (21) bildet, und jede der Seitenwände (23) am Übergang (24) von der ersten Öffnung (11) zu der zweiten Öffnung (21) eine Dicke aufweist, die kleiner ist, als ein Abstand (D) zwischen der Wurzel des Mundstückes (41) der Spritze (S) und dem Bodenteil der Nabe (44) der Nadel (43), wenn die Nadel (43) an der Spritze (S) befestigt ist, wobei jede der Seitenwände (23) zum geschlossenen Ende (25) der zweiten Öffnung (21) allmählich dicker wird, wobei die maximale Dicke der Seitenwände (23) größer ist, als der Abstand (D),
**dadurch gekennzeichnet, daß**
die Oberfläche (26) des Gleitteils (22) an der Rückseite des plattenförmigen Teils (10), auf der das Bodenteil der Nabe (44) gleitet, eine Fläche bildet, die sowohl in Richtung der ersten Öffnung (11) als auch in Richtung der zweiten Öffnung (21) geneigt ist, und daß eine zweite Nadelabzugsvorrichtung (30) vorgesehen ist, um eine einschraubbare Nadel herauszulösen,
wobei die zweite Nadelabzugsvorrichtung (30) eine dritte Öffnung (31) aufweist, die sich seitlich von der ersten Öffnung (11) erstreckt,
und die dritte Öffnung (31) einen Teil (32) aufweist, der so bemessen ist, daß die Nabe (44) der Nadel (43) darin eingeschoben werden kann, wobei der Teil (32) einen Vorsprung (34) aufweist, um die Nabe (44) der Nadel (43) daran zu hindern, sich zu drehen, wenn die Spritze (S) gedreht wird.

2. Vorrichtung nach Anspruch 1, **dadurch gekennzeichnet, daß** der Winkel zwischen beiden Kantenlinien jeder Seitenwand (23) in Längsrichtung der zweiten Öffnung (21) einen Wert von 5° bis 45° hat.

3. Vorrichtung nach Anspruch 1, **dadurch gekennzeichnet, daß** die dritte Öffnung (31) einen groß bemessenen Teil (32) aufweist, der eine solche Größe hat, daß die Nabe (44) der Nadel (43) leicht darin eingeschoben werden kann, sowie einen klein bemessenen Teil (33), der sich in vertikaler Richtung von dem groß bemessenen Teil (32) erstreckt und eine solche Größe aufweist, daß nur die Spitze der Nabe (44) darin eingeschoben werden kann, wobei der Vorsprung (34) an einem Teil der Seitenwand des klein bemessenen Teils (33) angeordnet ist.

4. Vorrichtung nach Anspruch 1,
   **dadurch gekennzeichnet, daß**
   der plattenförmige Teil (10) so ausgelegt ist, daß er löslich an einem Behälter für die Aufnahme von benutzten Injektionsnadeln befestigt werden kann.

5. Behälter für die Aufnahme von benutzten Injektionsnadeln, der folgendes aufweist:
   einen Behälter (B), und
   eine Injektionsnadelabzugsvorrichtung nach einem der vorausgegangenen Anprüche, die in einem Teil des Behälters angeordnet ist.

6. Behälter nach Anspruch 5, in dem die Injektionsnadelabzugsvorrichtung löslich in dem Behälter befestigt ist.

# FIG. 1

# FIG.2

# FIG.3

# FIG.4

# FIG.5

# FIG.6

# FIG.7

# FIG.8

FIG.9

FIG.10

FIG.11

FIG.12

FIG.13

# FIG. 14

FIG.15

FIG.16

# F I G . 17